# EUROPEAN PATENT APPLICATION

(11) **EP 1 681 013 A1**
(43) Date of publication of application: **19.07.2006**
(21) Application number: 05781405.5
(22) Date of filing: 02.09.2005
(51) Int. Cl.: A61B 1/00, G02B 23/24

(54) **ENDOSCOPE**

(30) Priority: 03.09.2004 JP 2004257575
(71) Applicant: Olympus Corporation, Tokyo 151-0072 (JP)
(72) Inventor: Eljiro Sato, Tokyo 192/8512 (JP); Kaoru Tsuruoka, Tokyo 192-8512 (JP)
(74) Representative: von Hellfeld, Axel
(86) International application number: PCT/JP2005/016139
(87) International publication number: WO 2006/025534

(57) **Abstract**

An endoscope (1) includes an insertion section (2) to be inserted from a distal end into a subject. The endoscope (1) further includes a bendable bend portion (12) disposed between the distal end and a proximal end of the insertion section, an actively bent region (L₁, L₁₁, L₁₂, L_{UD1}, L_{LR1}) which is disposed in the bend portion and which is to be bent in accordance with operator's operation, and a passively bent region (L₂, L₂₁, L₂₂, L_{UD2}, L_{LR2}) which is disposed in the bend portion and which is to be bent on receiving an external force.

## Description

### Technical Field

The present invention relates to an endoscope whose insertion section is provided with a bendable bend portion so that the bend portion is bent by an operation of an operation section.

### Background Art

An endoscope includes an insertion section to be inserted into a body cavity or the like. The insertion section includes a bendable bend portion. In general, the bend portion has a plurality of node rings as pieces to be bent, and adjacent node rings are successively rotatably attached. An operating wire connected to a distal end member of the bend portion can be moved in an axial direction to curve the total length of the bend portion.

The operating wire is passed from the operation section through a non-compressive densely-wound coil for wire guide disposed in a flexible tubular portion on a proximal end of the bend portion, and guide rings disposed on the node rings of the bend portion, and the wire is guided to the distal end member of the bend portion. Therefore, the operating wire can transmit an operating force of the operation section to a distal end of the bend portion. A distal end of the densely-wound coil is fixed to a distal end portion of the flexible tubular portion of the insertion section. Therefore, the operating force of the operating wire can be applied to the bend portion.

In Jpn. Pat. Appln. KOKAI Publication No. 11-155806, an endoscope is proposed in which only a distal end region portion of the bend portion is bent downwards with a small radius of curvature, and the whole bend portion is bent upwards with a large radius. In this endoscope, an interval between the adjacent node rings differs with the node rings arranged on the distal end of the bend portion and the node rings arranged on the proximal end. That is, rotatable amounts of the adjacent node rings differ in a case where the adjacent node rings abut on each other. Therefore, a bent amount of the bend portion is defined by each roatable amount.

As described above, in the conventional endoscope, the operating wire is guided to the distal end portion of the flexible tubular portion by the non-compressive densely-wound coil. The operating wire extends forwards from the distal end of the non-compressive densely-wound coil to the bend portion, and is connected to the distal end of the bend portion. A region forcibly bent by the operating wire exhibits a characteristic that a bent shape of the region is comparatively strongly maintained. A region just behind the bent portion has a linear state up to the following flexible tubular portion.

Therefore, in a case where the endoscope is inserted into a bent or small cavity, when the insertion section is pushed inwards while curving the bend portion, the bent portion abuts on a body cavity wall, an internal organ or the like. Therefore, the bend portion is susceptible to an external force from the body cavity wall, the internal organ or the like. Moreover, when the external force is applied to the bend portion, a stress is concentrated on a connecting portion between the bend portion and the flexible tubular portion, and a burden is imposed on the connecting portion. A burden is also imposed on the body cavity wall, the internal organ or the like which abuts on the bent portion.

### Disclosure of Invention

The present invention has been developed in view of the above-described problem, and an object is to provide an endoscope in which a burden due to a received external force is reduced in a case where a bend portion is bent and operated.

In an aspect of the present invention, an endoscope includes an insertion section which has a distal end and a proximal end and which is to be inserted from the distal end into a subject; a bendable bend portion disposed between the distal end and the proximal end of the insertion section; an actively bent region which is disposed in the bend portion and which is to be bent in accordance with operator's operation; and a passively bent region which is disposed in the bend portion and which is to be bent on receiving an external force.

In another aspect of the present invention, an endoscope includes an insertion section having a distal end and a proximal end; a bend portion disposed on a side of the distal end of the insertion section; at least one operating wire which is disposed in the insertion section and whose one end is connected to the bend portion; a wire connecting portion which connects the end of the operating wire to the bend portion to form, in the bend portion, an actively bent region to be bent and operated in accordance with operator's operation and a passively bent region to be bent on a proximal end side from the actively bent region by an external force; and a wire guide tube which is disposed in the bend portion on the proximal end side from the wire connecting portion and which is connected to a distal end of the wire connecting portion.

### Brief Description of Drawings

FIG. 1 is a schematic diagram showing the whole endoscope in a first embodiment of the present invention;
FIG. 2 is a schematic vertically sectional view showing a part of an insertion section of the endoscope in the first embodiment of the present invention;
FIG. 3 is a schematic perspective view showing that a distal end main body of the insertion section of the endoscope is connected to an outer member in the first embodiment of the present invention;
FIG. 4 is a schematic vertically sectional view of a modification schematically showing a distal end of the insertion section of the endoscope in the first embodiment of the present invention, and showing another mode to connect a channel tube to the distal end main body;
FIG. 5 is a schematic vertically sectional view of a tubular member of a flexible tubular portion in the insertion section of the endoscope in the first embodiment of the present invention;
FIG. 6 is a schematic perspective view showing a constitution of a core member of a bend portion in the insertion section of the endoscope in the first embodiment of the present invention;
FIG. 7 is a schematic diagram showing that the insertion section of the endoscope in the first embodiment of the present invention is disposed in a renal pelvis through an ureter;
FIG. 8 is a schematic perspective view showing a constitution of a core member of a bend portion in an insertion section of an endoscope in a second embodiment of the present invention;
FIG. 9 is a schematic perspective view showing a constitution of a core member of a bend portion in an insertion section of an endoscope in a third embodiment of the present invention;
FIG. 10 is a schematic perspective view showing a constitution of a core member of a bend portion in an insertion section of an endoscope in a fourth embodiment of the present invention;
FIG. 11 is a schematic perspective view showing a constitution of a core member of a bend portion in an insertion section of an endoscope in a fifth embodiment of the present invention;
FIG. 12 is a schematic vertically sectional view showing a constitution of a core member of a bend portion in an insertion section of an endoscope in a sixth embodiment of the present invention;
FIG. 13 is a schematic vertically sectional view showing a constitution of a core member of a bend portion in an insertion section of an endoscope in a seventh embodiment of the present invention;
FIG. 14 is a schematic diagram showing that an actively bent region of the bend portion of the insertion section is bent in the endoscope of the seventh embodiment of the present invention;
FIG. 15 is a schematic perspective view showing a constitution of a core member of a bend portion in an insertion section of an endoscope in an eighth embodiment of the present invention;
FIG. 16 is a schematic side view showing that an actively bent region of the bend portion of the insertion section is bent in the endoscope of the eighth embodiment of the present invention; and
FIG. 17 is a schematic plan view showing that the actively bent region of the bend portion of the insertion section is bent in the endoscope of the eighth embodiment of the present invention.

### Best Mode for Carrying Out the Invention

### <First Embodiment>

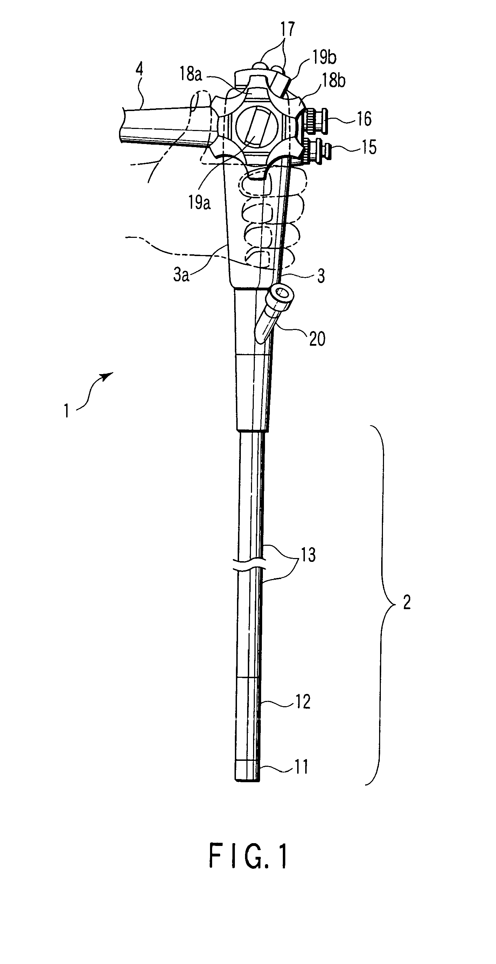

There will be described an electronic endoscope 1 in a first embodiment of the present invention with reference to FIGS. 1 to 8. The endoscope 1 is inserted into, for example, ureter, lung, ear or the like, except digestive canal, for use in performing observation or treatment. FIG. 1 schematically shows the whole endoscope 1. As shown in FIG. 1, the endoscope 1 includes an insertion section 2 to be inserted into a subject and an operation section 3 disposed on a proximal end of the insertion section 2. The operation section 3 is provided with a universal cord 4. A connector (not shown) is disposed on a distal end of the universal cord 4 extended from the operation section 3. The connector can be connected to a light source device and a video processor (not shown). Therefore, the endoscope 1 can be connected to the light source device and the video processor.

As shown in FIG. 1, the insertion section 2 includes: a hard distal end hard portion 11 disposed on the distal end of the insertion section; a bend portion 12 disposed on a rear end of the distal end hard portion 11; and a flexible tubular portion 13 disposed on the rear end of the bend portion 12 and having flexibility.

The operation section 3 of the endoscope 1 has a grasping portion 3a to be grasped in a case where an operator holds the endoscope 1. Above the grasping portion 3a, there are disposed an operation button 15 for air-feed and water-feed, and a suction operating button 16 to perform a suction operation. A head portion of the operation section 3 is provided with a plurality of remote switch buttons 17 to remove-operate the above-described video processor.

A pair of angle knobs 18a, 18b are rotatably disposed as operating members for use in curving the bend portion 12 of the insertion section 2 on side faces of the operation section 3. The angle knobs 18a, 18b are provided with brake members to lock the angle knobs 18a, 18b in operator's operated positions, respectively. In the present embodiment, the angle knob 18a is provided with a brake knob 19a. The angle knob 18b is provided with a brake lever 19b. It is to be noted that a treating instrument insertion port 20 is disposed under the grasping portion 3a.

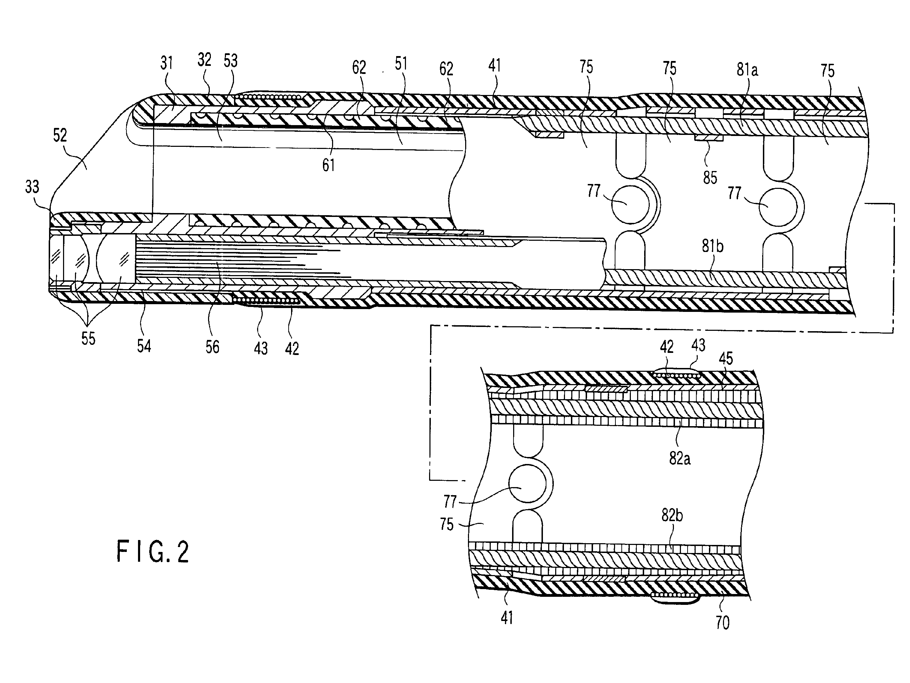

Next, there will be specifically described constitutions of the distal end hard portion 11, the bend portion 12, and the flexible tubular portion 13 in the insertion section 2. As shown in FIG. 2, the distal end hard portion 11 of the insertion section 2 includes a distal end main body 31, a distal end outer member 32, and a protruding portion 33. The distal end main body 31 is covered with the distal end outer member 32 made of a resin. The distal end outer member 32 is formed of a molded single component. The protruding portion 33 protruding forwards is formed under the distal end of the distal end outer member 32. That is, in this embodiment, the tip of the distal end outer member 32 does not cross an axial direction of the insertion section 2 at right angles, and the tip is obliquely formed.

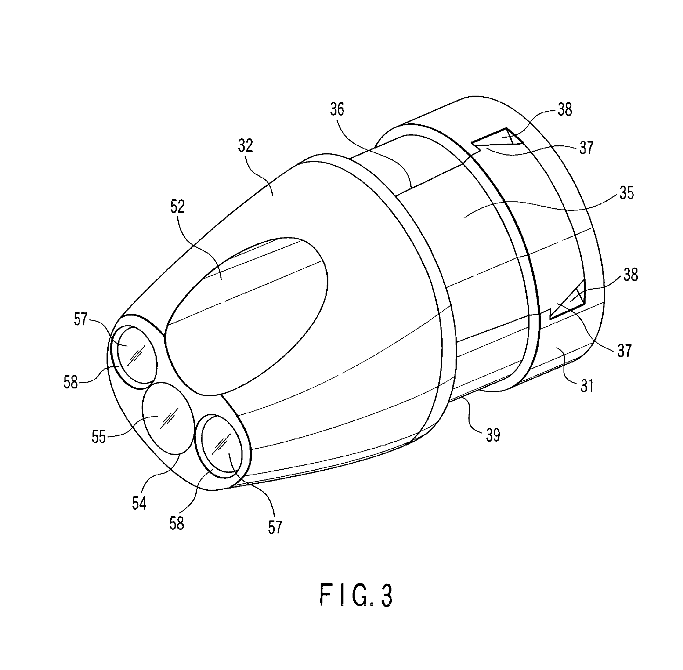

As shown in FIG. 3, two lingual pieces (protrusions) 35 extending rearwards are integrally formed on opposite sides of a rear end of the distal end outer member 32. These lingual pieces 35 are molded together with the distal end outer member 32 during molding of the distal end outer member 32. These lingual pieces 35 are fitted into a concave portion 36 formed into a shape corresponding to an outer peripheral side surface portion of the distal end main body 31, and engaged with the distal end main body 31. Engaging portions 37 are formed on opposite sides of an extended end portion of the lingual piece 35. In the concave portion 36, recessed portions 38 are formed in which the engaging portions 37 are inserted and engaged.

When the distal end main body 31 is connected to the distal end outer member 32, a pair of left and right lingual pieces 35 are expanded sideways to fit the lingual pieces 35 into the concave portion 36. Then, as shown in FIG. 3, the distal end main body 31 is connected to the distal end outer member 32. In this manner, an engagement mechanism is adopted to connect the distal end main body 31 to the distal end outer member 32. Therefore, fixing components such as screws are unnecessary in connecting the distal end main body 31 to the distal end outer member 32, and the number of the components can be reduced. The outer member 32 can be easily and securely attached to the distal end main body 31, and assembled. Moreover, it is possible to reduce the number of assembling steps and assembling time for this unit, thus an assembling property of the endoscope 1 can be improved.

It is to be noted that there are no restrictions on shapes of the lingual pieces 35 and the concave portion 36 into which the pieces are fitted as long as they can be engaged with each other. Various configurations can be considered, for example, a distal end portion of the lingual piece 35 is gradually enlarged, or the engaging portion 37 is formed on one side only. One or more lingual pieces 35 are sufficient.

As shown in FIG. 3, in a state in which two lingual pieces 35 of the distal end outer member 32 are fitted into the concave portion 36 of the distal end main body 31, an annular groove portion 39 is formed in the whole periphery of a rear end outer peripheral face of the distal end main body 31 including the lingual pieces 35. As shown in FIG. 2, the groove portion 39 is covered with a distal end edge portion of a coating member 41 which forms an envelope of the bend portion 12 described later. An outer periphery of the distal end edge portion of the coating member 41 is tightened onto the annular groove portion 39 with thread 42. Moreover, an adhesive 43 is applied and solidified to the thread tightening portion. In this manner, the coating member 41 is fixedly connected to the distal end hard portion 11.

The coating member 41 is formed to be thin in order to reduce an outer diameter of the bend portion 12. The coating member 41 improves a tear strength and a tensile strength in order to improve a resistance to expansion and contraction and a resistance to perforation during the curving. In the present embodiment, a material such as thermoplastic polyurethane elastomer (E580) is used instead of a heretofore generally used coating member made of a fluorine-based rubber.

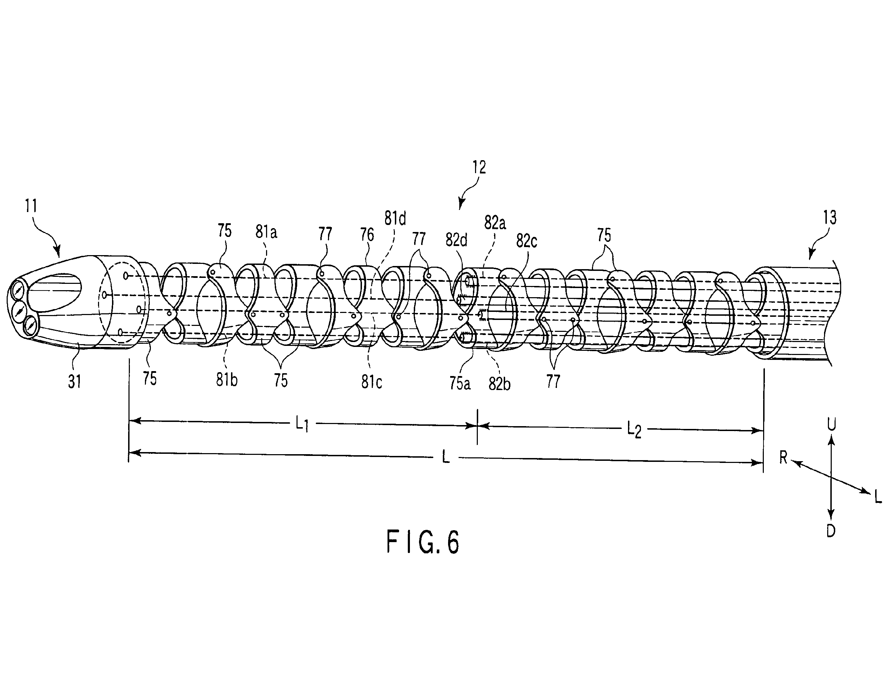

As described above, the distal end of the coating member 41 is fixed to the distal end main body 31 by tightening the thread. A rear end of the coating member 41 is also connected and fixed to a connecting tube 45 disposed on a distal end portion of the flexible tubular portion 13 by thread tightening and bonding as described above. In the present embodiment, a middle portion of the coating member 41 to cover a core member (node ring 75) of the bend portion 12 is fixedly connected to, for example, a node ring 75a (see FIG. 6) of the bend portion 12 positioned corresponding to the middle portion as described later. Therefore, the middle portion of the coating member 41 is formed to be prevented from being displaced from the node ring 75a. Therefore, even if the bend portion 12 is repeatedly bent using the thin extensible coating member 41, the coating member 41 is prevented from being largely displaced forwards and backwards over the total length.

As shown in FIGS. 2 and 3, the distal end hard portion 11 of the insertion section 2 is provided with a hole 53 which forms a channel 51 and a port portion 52. A hole 54 to dispose a member of an objective optical system for observation is formed under the hole 53. Objective lenses 55 and an image guide fiber bundle 56 are disposed in the hole 54. On opposite sides of the hole 54, holes 58 are formed to dispose illuminating optical system members such as illuminative lenses 57 or a light guide fiber bundle (not shown).

As shown in FIG. 2, a hole 61 having a diameter larger than that of another portion is formed in an inner portion (rear end portion) of the hole 53 which forms the channel 51 in the distal end main body 31. A distal end edge portion of a channel tube 62 as a duct member forming the channel 51 is fitted into the hole 61. A distal end edge portion of the channel tube 62 is connected and fixed to the distal end main body 31 by bonding or the like. The channel tube 62 is guided to the operation section 3 through the bend portion 12 and the flexible tubular portion 13 of the insertion section 2, respectively. A proximal end of the channel tube 62 is connected to a cap of the treating instrument insertion port 20 of the operation section 3.

In the channel tube 62, a flexible core member is used such as a braid formed by braiding or plaiting a metal wire, or a flex made of a metal coil. Such core member is sandwiched between soft inner and outer resin layers. That is, the channel tube 62 has a sandwich structure. Such channel tube 62 has a structure in which the core member is sandwiched between the resin layers. Therefore, buckling or kinking is not easily generated in the channel tube 62. Since a pitch of a material of the core member sandwiched between the resin layers of the channel tube 62 is reduced, flexibility of a duct member can be secured.

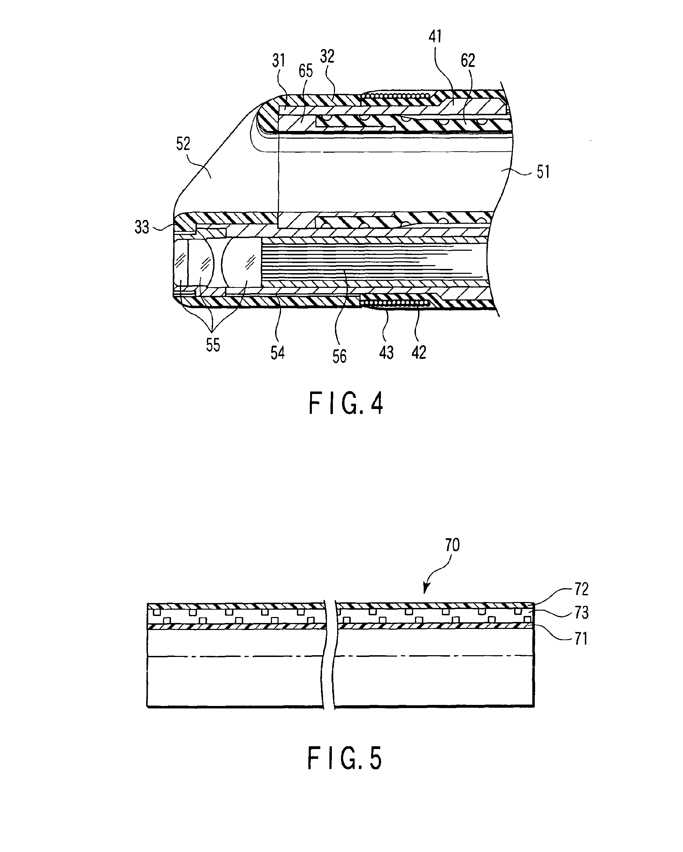

FIG. 4 shows a modification showing another mode to connect the above-described channel tube 62 to the distal end main body 31. That is, the distal end main body 31 is provided with a connecting cylindrical member 65 disposed coaxially with the channel 51. A distal end of the channel tube 62 is fitted into an outer periphery of the connecting cylindrical member 65, and the distal end is fixedly connected to the outer periphery. An outer diameter of a fitted portion of the connecting cylindrical member 65 is formed to be larger than an inner diameter of the above-described channel tube 62. In a case where the distal end portion of the channel tube 62 is fitted into the outer periphery of the connecting cylindrical member 65, the diameter of the distal end portion of the channel tube 62 is enlarged, and the portion is fitted into the outer periphery of the connecting cylindrical member 65.

In addition, in a case where the core member is sandwiched between the resin layers in this type of channel tube 62, it is generally difficult to enlarge the diameter of the channel tube 62. In the present embodiment, a wire pitch of a core member such as the braid is formed to be larger than that of another portion in the distal end portion of the channel tube 62 which is fitted into the outer periphery of the connecting cylindrical member 65, or the wire of the core member is removed. Therefore, the diameter of the distal end portion of the channel tube 62 to be fitted into the outer periphery of the connecting cylindrical member 65 is easily enlarged. Accordingly, the distal end portion of the channel tube 62 can be easily fitted into the outer periphery of the connecting cylindrical member 65 while applying heat to the distal end portion of the channel tube 62 to be fitted into the outer periphery of the connecting cylindrical member 65, and enlarging the diameter of the distal end portion of the channel tube 62. The distal end portion of the channel tube 62 fitted into the outer periphery of the connecting cylindrical member 65 is fixed to the connecting cylindrical member 65 by bonding and/or thread tightening. After fixing the portion to the member in this manner, the connecting cylindrical member 65 connected to the channel tube 62 may be attached to the distal end main body 31.

The flexible tubular portion 13 of the insertion section 2 may be formed by a tubular member 70 as shown in FIG. 5. The tubular member 70 includes an inner layer resin 71, an outer layer resin 72, and a core member 73 sandwiched between the resin layers 71 and 72. In the core member 73, a material is used such as a material formed by braiding or plaiting a metal wire or a material made of a metal flex coil. A slightly harder material is used in both of the resin layers 71 and 72 in order to improve a twist follow-up property and a resistance to collapse of the flexible tubular portion 13. When the wire pitch of the core member 73 is set to be smaller, softness of the flexible tubular portion 13 is kept.

Next, there will be described a structure of the bend portion 12 of the insertion section 2. As shown in FIG. 6, the bend portion 12 includes, as the core member, a bend main body 76 having a substantially tubular shape as a whole and constituted of a plurality of node rings (pieces to be bent) 75. An outer periphery of the bend main body 76 is covered with the above-described coating member 41 (see FIGS. 2 and 4). The node rings 75 constituting the bend main body 76 are arranged in a row in a longitudinal axis direction of the insertion section 2 (bend portion 12). Adjacent ends of these node rings 75 are rotatably attached alternately or every other end by rivet-like pins (support portions) 77 in a horizontal or vertical position. Therefore, the node rings 75 are rotatable centering on the pins 77, respectively, in an up and down (UD) direction or a left and right (LR) direction. Therefore, the whole bend main body 76 can be bent in two and/or four directions (the up and down directions and/or the left and right directions) on the basis of a state in which the bend portion 12 is substantially straightened out.

As shown in FIG. 6, the leading-edge node ring 75 is fixedly connected to the distal end main body 31 in the distal end hard portion 11 of the insertion section 2. As shown in FIG. 2, the rearmost-end node ring 75 is fixedly connected to the distal end of the flexible tubular portion 13 of the insertion section 2 directly or via the connecting tube 45.

In the insertion section 2, four operating wires 81a, 81b, 81c, and 81d are passed through the flexible tubular portion 13 and the bend portion 12 in order to curve the bend portion 12 in the up and down directions and/or the left and right directions. Any of distal ends of these operating wires 81a, 81b, 81c, and 81d is fixedly connected to a distal end portion of the bend portion 12, such as the leading-edge node ring 75 or the distal end main body 31. Distal end fixing portions of four operating wires 81a, 81b, 81c, and 81d are disposed in positions substantially corresponding to the rotation centers of the up and down directions and/or the left and right directions, respectively.

Hand sides of the operating wires 81a, 81b, 81c, and 81d individually correspond to wire guide tubes 82a, 82b, 82c, and 82d having flexibilities, respectively. The operating wires 81a, 81b, 81c, and 81d are guided into the operation section 3 through the wire guide tubes 82a, 82b, 82c, and 82d. Each of the wire guide tubes 82a, 82b, 82c, and 82d is formed by, for example, a densely wound coil having flexibility. The wire guide tubes 82a, 82b, 82c, and 82d individually guide axial-direction movements of the operating wires 81a, 81b, 81c, and 81d, respectively. Therefore, curving and operating forces of the operating wires 81a, 81b, 81c, and 81d are transmitted to the bend portion 12.

The operating wires 81a, 81b, 81c, and 81d protruding from the distal ends of the wire guide tubes 82a, 82b, 82c, and 82d are guided forwards through guide rings disposed in the node rings 75 arranged in front, respectively (see FIG. 2).

In a case where the bend portion 12 of the insertion section 2 is bent, a wire moving and operating device (not shown) incorporated in the operation section 3 is operated by the angle knobs 18a, 18b of the operation section 3. At this time, in either a set of the upper and lower operating wires 81a, 81b or a set of the left and right operating wires 81c, 81d, the operating wires are moved and operated in opposite directions, respectively.

As shown in FIG. 6, the distal ends of the wire guide tubes 82a, 82b, 82c, and 82d are fixedly attached to an inner face of the middle node ring 75a in a group of the node rings 75 of the bend main body 76. That is, the distal ends of the wire guide tubes 82a, 82b, 82c, and 82d are fixed to the node ring 75a in the middle of the bend main body 76. Therefore, the curving and operating forces of the operating wires 81a, 81b, 81c, and 81d are concentrated on the node rings 75 of a region L₁ before the node ring 75a. The region L₁ is an actively bent region in a region of the bend portion 12. The actively bent region L₁ is a portion to be bent in accordance with operator's rotating operation (curving operation) of the angle knobs 18a, 18b.

That is, the curving and operating forces of the operating wires 81a, 81b, 81c, and 81d are exerted on the actively bent region L₁ positioned only on the distal end side in a range of the whole region L as the bend portion 12 of the insertion section 2. On the other hand, the curving and operating forces of the operating wires 81a, 81b, 81c, and 81d are hardly exerted on a proximal end region L₂. That is, the proximal end region L₂ of the bend portion 12 is a so-called non-bent region that cannot be bent by the operating wires 81a, 81b, 81c, and 81d. Therefore, the only distal end region L₁ of the bend portion 12 is positively bent.

The proximal end region L₂ of the bend portion 12 is bent in a case where a comparatively large external force is applied to the region itself or the distal end portion of the insertion section 2 including the bend portion 12. That is, the proximal end region L₂ forms a non-bent region that cannot be bent by the operating wires 81a, 81b, 81c, and 81d. The non-bent region is a passively bent region which can be bent under the external force. The curving and operating forces of the operating wires 81a, 81b, 81c, and 81d hardly act on the passively bent region L₂. Therefore, flexibility of the region L₂ is higher than that of the actively bent region L₁ when bent, and the region is easily bent by the external force. The flexibility of the region L₂ is higher than that of the flexible tubular portion 13. In other words, a portion (easily bent portion) having a flexibility higher than that of the flexible tubular portion 13 is disposed between the actively bent region L₁ and the flexible tubular portion 13.

Bendability of this proximal end region L₂ overcomes the flexibility of the flexible tubular portion 13 disposed on a rear end of the bend portion 12. The region has a flexible constitution such that the proximal end region L₂ of the bend portion 12 starts to curve before the flexible tubular portion 13 starts to bend in a case where the external force is applied to the distal end side of the flexible tubular portion 13. In general, on receiving the external force, the proximal end region L₂ of the bend portion 12 is bent more easily (flexibility is higher) than the flexible tubular portion 13 on the proximal end of the proximal end region L₂. The flexible tubular portion 13 resists bending owing to the external force rather than the proximal end region L₂ of the bend portion 12. Furthermore, when the distal end hard portion 11 and the bend portion 12 of the insertion section 2 are horizontally floated, the proximal end region L₂ of the bend portion 12 is not bent or deflected owing to the weight of the distal end hard portion 11 and the bend portion 12. That is, the proximal end region L₂ of the bend portion 12 has such a strength that the distal end hard portion 11 does not sag owing to gravity.

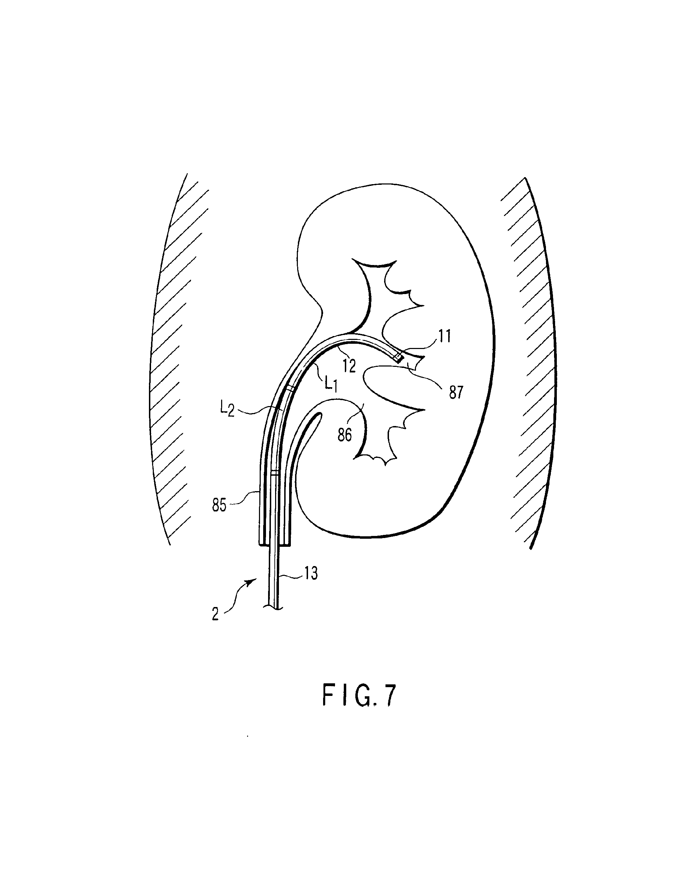

Next, there will be described a use example and a function in a case where the endoscope 1 is used. An operator appropriately curves the bend portion 12 while viewing an observation image displayed in a monitor, and inserts the insertion section 2 into a body cavity. FIG. 7 shows an example of a state in which the insertion section 2 is introduced into the body cavity. Specifically, this indicates that the insertion section 2 is led from a bladder (not shown) through a ureter 85 into a renal pelvis 86. In this case, while the distal end region L₁ of the bend portion 12 is bent, the insertion section 2 is pushed into the body cavity in order to bring the distal end hard portion 11 of the insertion section 2 close to calices 87.

In a case where the insertion section 2 is pushed inwards, since the distal end region L₁ is bent, the distal end hard portion 11 of the insertion section 2 is sometimes pressed onto an inner wall of the renal pelvis 86. Therefore, an external force is applied to the proximal end region L₂ of the bend portion 12 following the distal end region L₁. Usually, the insertion section 2 is pressed onto the internal organs, which imposes a burden. When a reactive force is exerted from the inner wall of the renal pelvis 86, the external force is applied to the thin insertion section 2, and a stress is concentrated on a connection end of the flexible tubular portion 13 connected to the bend portion 12 of the thin insertion section 2.

However, in the present embodiment, the proximal end region L₂ of the bend portion 12 is comparatively soft. Moreover, the proximal end region L₂ of the bend portion 12 is subsequently bent by the external force, and the external force is released. Therefore, any burden is prevented from being imposed on the renal pelvis 86, and the burden imposed on the thin insertion section 2 is also reduced.

### <Second Embodiment>

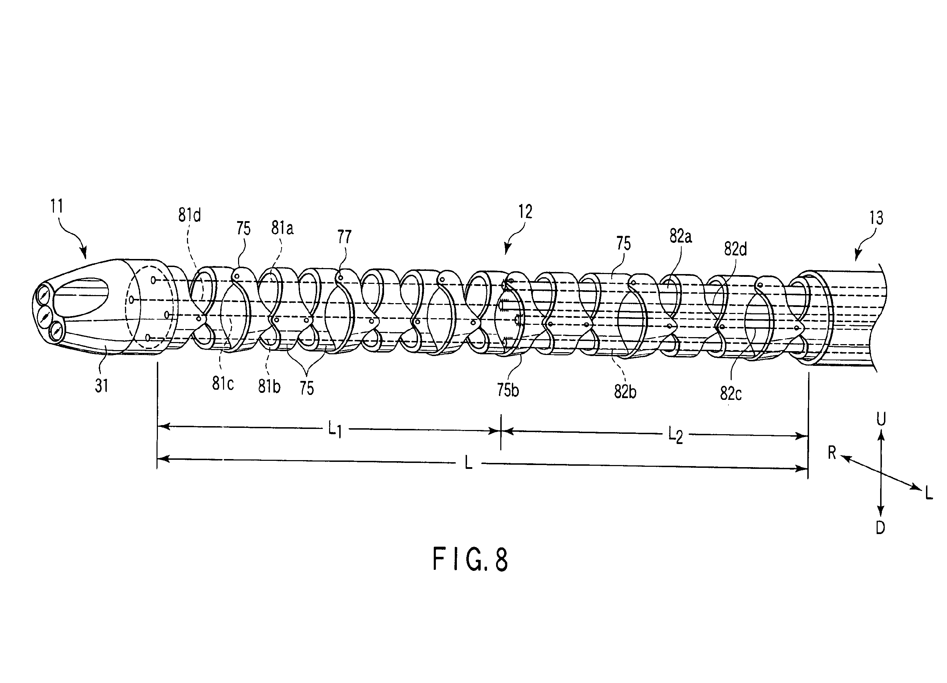

There will be described an endoscope 1 in a second embodiment of the present invention with reference to FIG. 8. This embodiment is a modification of the first embodiment, and the same members as those described in the first embodiment or members having the same functions as those of the first embodiment are denoted with the same reference numerals, and a detailed description is omitted. This also applies to third to eighth embodiments.

The endoscope 1 of the present embodiment is different from that of the above first embodiment in a structure of a core member of a bend portion 12 in an insertion section 2.

In the first embodiment, as to node rings 75 of a bend main body 76, the node rings 75 having substantially similar shapes and lengths are repeatedly arranged from a distal end to a proximal end of the bend portion 12. In the bend main body 76 of the present embodiment, an arrangement pitch of the node rings 75 of a proximal end region L₂ is set to be longer than that of the node rings 75 of an actively bent region L₁. That is, an axial-direction length of each node ring 75 of the proximal end region L₂ is formed to be longer than that of each node ring 75 of the actively bent region L₁. Therefore, the actively bent region L₁ can be bent with a smaller radius as compared with the proximal end region L₂. On the other hand, the proximal end region L₂ is suitable for use in a case where the region does not have to be bent with the small radius.

### <Third Embodiment>

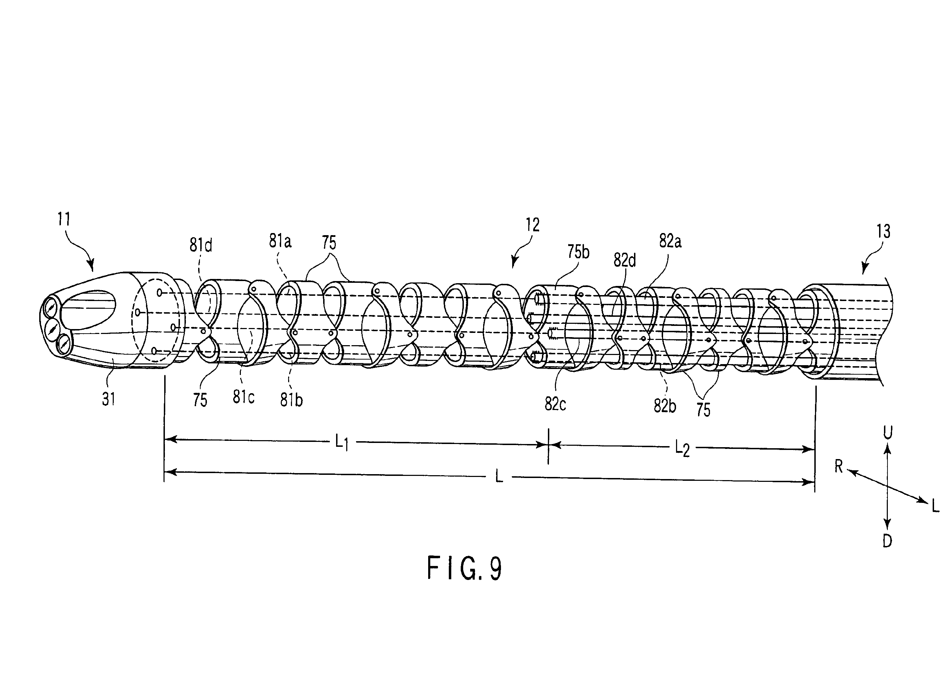

There will be described an endoscope 1 in a third embodiment of the present invention with reference to FIG. 9. In the endoscope 1 of the present embodiment, contrary to the above second embodiment, an arrangement pitch of node rings 75 of a proximal end region L₂ is set to be shorter than that of node rings 75 of an actively bent region L₁. That is, an axial-direction length of each node ring 75 of the proximal end region L₂ is formed to be shorter than that of each node ring 75 of the actively bent region L₁. Therefore, even when the total length of the proximal end region L₂ is shortened, the region is bent with a small radius, and an external force can be absorbed in a small region. Therefore, the present embodiment is suitable for use in a small body cavity.

### <Fourth Embodiment>

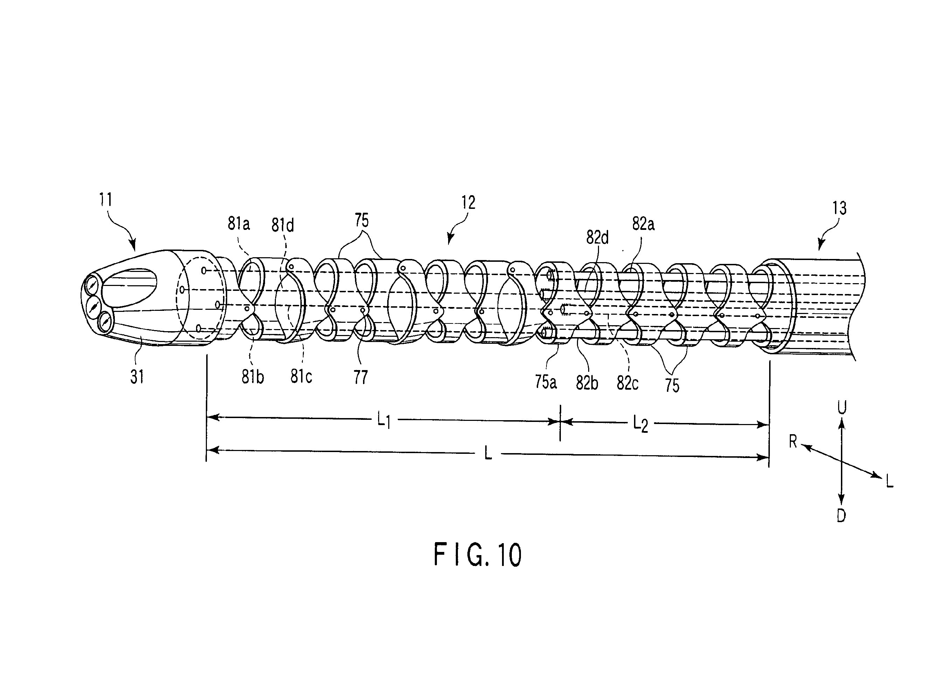

There will be described an endoscope 1 in a fourth embodiment of the present invention with reference to FIG. 10. In the endoscope 1 of the present embodiment, a plurality of node rings 75 in a proximal end region L₂ are connected to each other via pins 77 so that the rings rotate in only two directions (up and down directions in the present embodiment) on the basis of a substantially straightened state of a bend portion 12. The node rings do not rotate in four directions of the up and down directions and left and right directions. Bending directions of the proximal end region L₂ are set along two directions (up and down directions) in which the rings are actually most frequently bent. Accordingly, a constitution of the bend portion 12 can be simplified.

### <Fifth Embodiment>

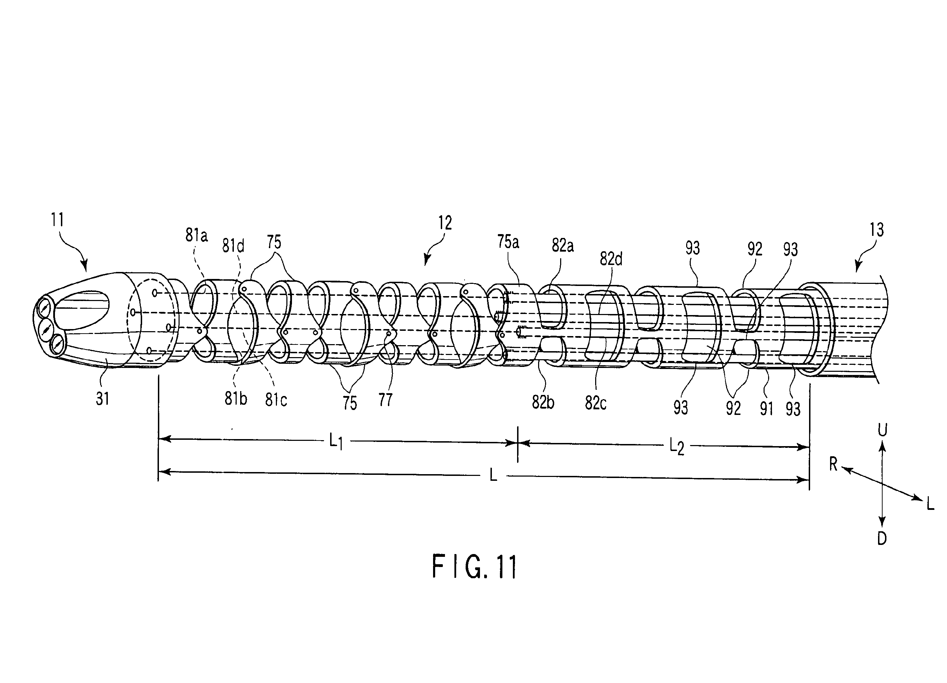

There will be described an endoscope 1 in a fifth embodiment of the present invention with reference to FIG. 11. In the endoscope 1 of the present embodiment, instead of the above-described node rings 75 (see FIGS. 6 and 8 to 10), an elastic material formed by working a cylindrical metal member 91 and having a flexibility is used in a core member of a proximal end region L₂ of a bend portion 12. A plurality of cut portions 92 are formed in the cylindrical metal member 91. Remaining connecting portions 93 due to these cut portions 92 constitute rotation supports of the proximal end region L₂ of the bend portion 12. The proximal end region L₂ of the bend portion 12 can be elastically bent as a whole in up and down directions and/or left and right directions. According to the present embodiment, it is possible to simplify a structure of a bend core member in the proximal end region L₂ of the bend portion 12.

It is to be noted that the core member in the proximal end region L₂ of the bend portion 12 is not limited to the above-described metal member 91. There may be used a flex member which is bendable under an external force and which has flexibility, such as a material obtained by covering a spirally wound flex coil member with a braid.

### <Sixth Embodiment>

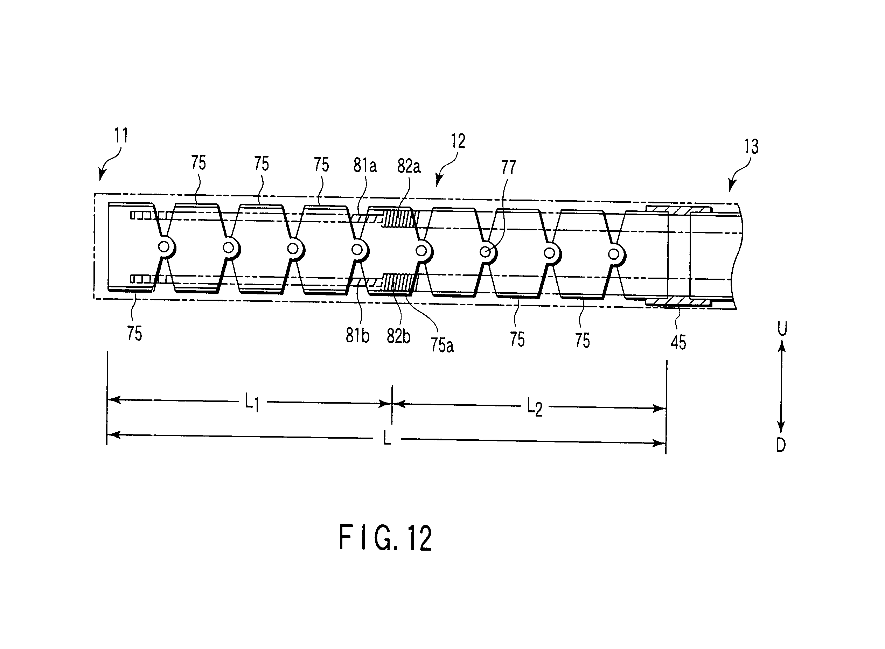

There will be described an endoscope 1 in a sixth embodiment of the present invention with reference to FIG. 12. In the present embodiment, node rings 75 of a bend main body 76 are connected to one another so that the rings can rotate in up and down directions only. Therefore, the endoscope includes: upper and lower operating wires 81a, 81b which curve the bend main body in the up and down directions; and upper and lower wire guide tubes 82a, 82b which guide the wires. Since the endoscope can be operated in the same manner as in the first embodiment except an operation in left and right directions, components are denoted with the same reference numerals, and detailed description thereof is omitted.

### <Seventh Embodiment>

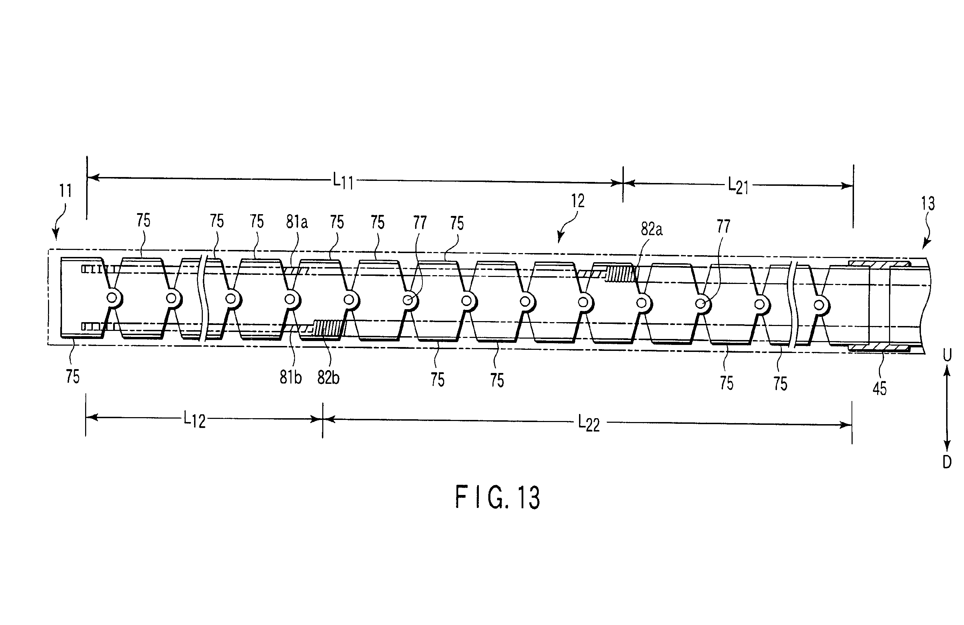

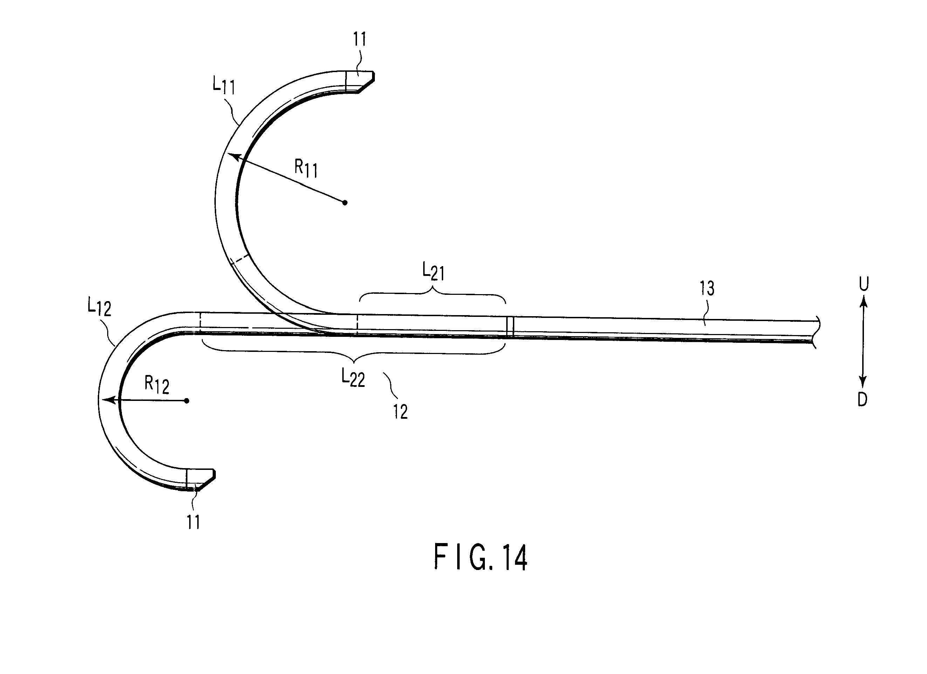

There will be described an endoscope 1 in a seventh embodiment of the present invention with reference to FIGS. 13 and 14. The present embodiment is similar to the above-described sixth embodiment in that positions where distal ends of upper and lower wire guide tubes 82a, 82b are fixed to node rings 75 are displaced forwards and backwards in an axial direction of a bend portion 12. Therefore, an actively bent region L₁ includes a first actively bent region L₁₁ and a second actively bent region L₁₂. A passively bent region L₂ includes a first passively bent region L₂₁ and a second passively bent region L₂₂.

The first actively bent region L₁₁ is bent by an upper operating wire 81a guided by the upper wire guide tube 82a whose distal end is fixed behind the lower wire guide tube 82b. The second actively bent region L₁₂ is bent by the lower operating wire 81b guided by the lower wire guide tube 82b whose distal end is fixed before the upper wire guide tube 82a. Therefore, as shown in FIG. 14, the actively bent regions L₁₁ and L₁₂ have different radii R₁₁, R₁₂ of curvatures. The radius R₁₁ of curvature of the first actively bent region L₁₁ is bent less than the radius R₁₂ of curvature of the second actively bent region L₁₂. Therefore, the radius R₁₁ of curvature of the first actively bent region L₁₁ is larger than the radius R₁₂ of curvature of the second actively bent region L₁₂.

Moreover, the first passively bent region L₂₁ is formed on a rear end of the first actively bent region L₁₁. The second passively bent region L₂₂ is formed on a rear end of the second actively bent region L₁₂. That is, even when rear end positions of the actively bent regions L₁₁, L₁₂ are displaced forwards and backwards in a region of the bend portion 12, the actively bent regions L₂₁, L₂₂ are immediately connected without any large intervals from rear ends of the actively bent regions, respectively.

### <Eighth Embodiment>

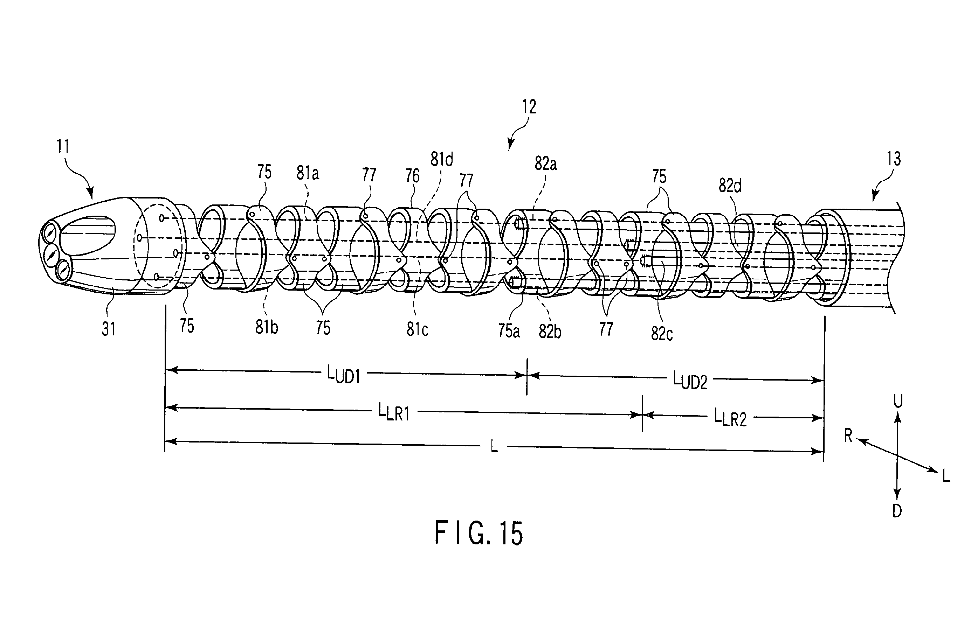

There will be described an endoscope 1 in an eighth embodiment of the present invention with reference to FIGS. 15 to 17. In the present embodiment, a position where distal ends of upper and lower wire guide tubes 82a, 82b are fixed to a node ring 75 is displaced from a position where distal ends of left and right wire guide tubes 82c, 82d are fixed to a node ring 75 forwards and backwards in an axial direction of a bend portion 12 in the same manner as in the first embodiment shown in FIG. 6.

An actively bent region L₁ includes a first actively bent region L_{UD1} and a second actively bent region L_{LR1}. An actively bent region L₂ includes a first passively bent region L_{UD2} and a second passively bent region L_{LR}2-

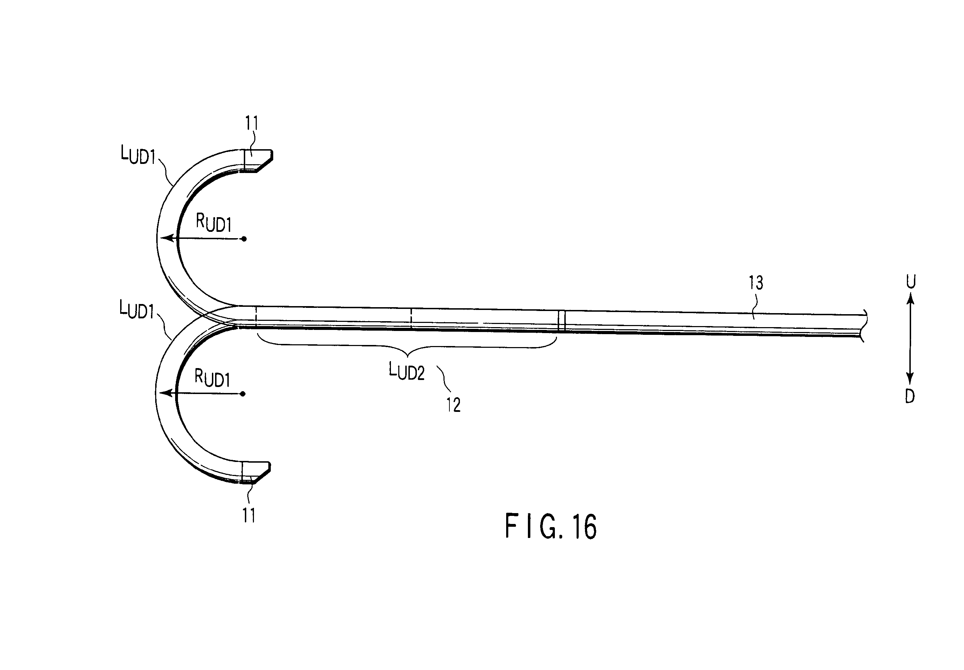

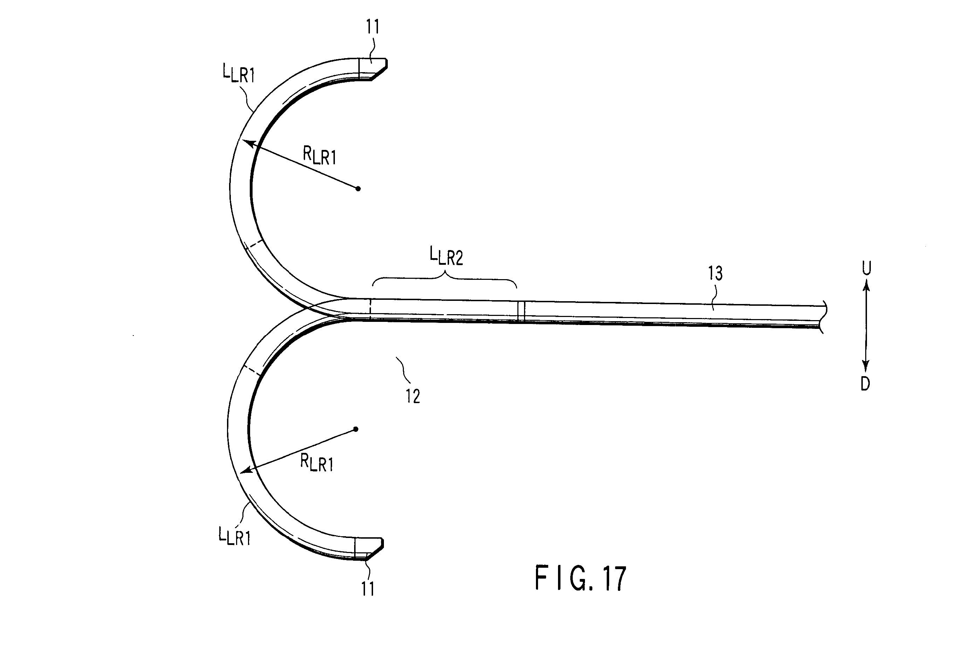

Here, as shown in FIG. 15, distal ends of left and right wire guide tubes 82c, 82d are fixed to a node ring 75 before a node ring 75 to which distal ends of the upper and lower wire guide tubes 82a, 82b. Therefore, the first actively bent region L_{UD1} is bent by an upper operating wire 81a and a lower operating wire 81b guided by the upper and lower wire guide tubes 82a, 82b positioned on a distal end side, respectively. A radius R_{UD1} of curvature of the first actively bent region L_{UD1} is shorter than a radius R_{LR1} of curvature (see FIG. 17) described later as shown in FIG. 16. At this time, a second passively bent region L_{UD2} is longer than a second passively bent region L_{LR2} (see FIG. 15). Moreover, as shown in FIG. 17, radii R_{LR1} of curvatures of the first actively bent regions L_{LR1} bent by left and right operating wires 81c, 81d guided by left and right wire guide tubes 82c, 82d positioned in a rear end, respectively, are longer than the radii R_{UD1} of curvatures (see FIG. 16). At this time, the second passively bent region L_{LR2} is shorter than the second passively bent region L_{UD2}. In any case, even when rear end positions of the actively bent regions L_{UD1}, L_{LR1} are displaced forwards and backwards in a region of the bend portion 12, the passively bent regions L_{UD2}, L_{LR2} are connected to rear ends of the regions.

It is to be noted that the present invention is not limited to the above embodiments. For example, sets of the operating wires and the wire guide tubes may be increased or decreased. A distal end fixing position of the wire guide tube may be appropriately selected. Furthermore, the present invention is applicable even to a fiber scope in which an image guide fiber bundle is used. In the fiber scope, a low pass filter is disposed in an emission end of the image guide fiber bundle facing an eyepiece lens of an eyepiece section, or the low pass filter may be disposed in an eyepiece lens group. When the low pass filter is incorporated in this manner, an impression of mesh by fiber strands of the image guide fiber bundle can be reduced. The above embodiment is an example applied to the endoscope 1 whose insertion section has a small diameter, such as an urinary endoscope, but the present invention is applicable to another type of endoscope.

Several embodiments have been specifically described with reference to the drawings, but the present invention is not limited to the above-described embodiments, and includes all embodiments performed without departing from the scope.

### Industrial Applicability

According to the embodiments described above, in a case where an external force is applied at a time when a bend portion is bent and operated, since a region behind the bent portion bends owing to the external force, it is possible to reduce burdens imposed on an insertion section, a body cavity wall, an internal organ and the like.

## Claims

1. An endoscope (1) **characterized by** comprising:
an insertion section (2) which has a distal end and a proximal end and which is to be inserted from the distal end into a subject;
a bendable bend portion (12) disposed between the distal end and the proximal end of the insertion section;
an actively bent region (L₁; L₁₁, L₁₂; L_{UD1}, L_{LR1}) which is disposed in the bend portion and which is to be bent in accordance with operator's operation; and
a passively bent region (L₂; L₂₁, L₂₂; L_{UD2}, L_{LR2}) which is disposed in the bend portion and which is to be bent on receiving an external force.

2. The endoscope (1) according to claim 1, **characterized in that** the insertion section (2) includes a flexible tubular portion (13) between the bend portion (12) and the proximal end, and
the passively bent region (L_{2;} L₂₁, L₂₂; L_{UD2,} L_{LR2}) has a flexibility which is higher than that of the flexible tubular portion.

3. The endoscope (1) according to claim 1 or 2, **characterized by** further comprising:
a plurality of operating wires (81a, 81b, 81c, 81d) whose distal ends are connected to the bend portion (12) and which are movable along axial directions thereof in accordance with the operator's operation; and
a plurality of wire guide tubes (82a, 82b, 82c, 82d) which is disposed in the insertion section (2) and through which the operating wires are inserted,
the actively bent region (L₁; L₁₁, L₁₂; L_{UD1}, L_{LR1}) including:
wire connecting portions (31, 75) connected to distal ends of the operating wires, respectively; and
wire guide tube connecting portions (75a) disposed on a proximal end side of the wire connecting portions and connected to distal ends of the wire guide tubes, respectively.

4. The endoscope (1) according to claim 3, **characterized in that** at least one of the wire guide tube connecting portions (75a) is disposed in a position different from that of another wire guide tube connecting portion with respect to a longitudinal axis direction of the bend portion (12).

5. The endoscope (1) according to any one of claims 1 to 4, **characterized in that** each of the actively bent region (L₁; L₁₁, L₁₂; L_{UD1}, L_{LR1}) and the passively bent region (L₂; L₂₁, L₂₂; L_{UD2}, L_{LR2}) includes:
a plurality of pieces (75) to be bent which are arranged in a row in the longitudinal axis direction of the bend portion (12); and
support portions (77) which support the pieces to be bent disposed adjacent to one another.

6. The endoscope (1) according to claim 5, **characterized in that** the pieces (75) to be bent have different lengths of the bend portion (12) in the longitudinal axis direction in the actively bent region (L₁; L₁₁, L₁₂; L_{UD1}, L_{LR1}) and the passively bent region (L2; L21, L₂₂; L_{UD2}, L_{LR2}).

7. An endoscope (1) **characterized by** comprising:
an insertion section (2) having a distal end and a proximal end;
a bend portion (12) disposed on a side of the distal end of the insertion section;
at least one operating wire (81a, 81b, 81c, 81d) which is disposed in the insertion section and whose one end is connected to the bend portion;
a wire connecting portion (31, 75) which connects the end of the operating wire to the bend portion to form, in the bend portion, an actively bent region (L₁; L₁₁, L₁₂; L_{UD1}, L_{LR1}) to be bent and operated in accordance with operator's operation and a passively bent region (L₂; L₂₁, L₂₂; L_{UD2}, L_{LR2}) to be bent on a proximal end side from the actively bent region by an external force; and a wire guide tube (82a, 82b, 82c, 82d) which is disposed in the bend portion on the proximal end side from the wire connecting portion and which is connected to a distal end of the wire connecting portion.

8. The endoscope (1) according to claim 7, **characterized by** further comprising:
a flexible tubular portion (13) disposed on the proximal end side of the insertion section (2) from the bend portion (12),
wherein the passively bent region (L₂; L₂₁, L₂₂; L_{UD2}, L_{LR2}) has a flexibility which is higher than that of the flexible tubular portion.

9. The endoscope (1) according to claim 7 or 8, **characterized by** further comprising:
a plurality of operating wires (81a, 81b, 81c, 81d); and
a plurality of wire guide tubes (82a, 82b, 82c, 82d) through which the operating wires are inserted,
at least one of the wire guide tube connecting portions (75a) having a different distance from the distal end of the insertion section as compared with another wire guide tube connecting portion.

10. The endoscope (1) according to any one of claims 7 to 9, **characterized in that** each of the actively bent region (L₁; L₁₁, L₁₂; L_{UD1}, L_{LR1}) and the passively bent region (L₂; L₂₁, L₂₂; L_{UD2}, L_{LR2}) includes:
a plurality of pieces (75) to be bent which are arranged in a row in the longitudinal axis direction of the bend portion (12); and
support portions (77) which support the pieces to be bent disposed adjacent to one another.

11. The endoscope (1) according to any one of claims 7 to 9, **characterized in that** the actively bent region (L₁; L₁₁, L₁₂; L_{UD1}, L_{LR1}) has a plurality of node rings (75) and support portions (77) which support the node rings disposed adjacent to one another, and
the passively bent region (L₂; L₂₁, L₂₂; L_{UD2}, L_{LR2}) includes an elastic member (91) having such a flexibility that the member is bendable on receiving an external force.

12. The endoscope (1) according to claim 10 or 11, **characterized in that** the actively bent region (L₁; L₁₁, L₁₂; L_{UD1}, L_{LR1}) and the passively bent region (L₂; L₂₁, L₂₂; L_{UD2}, L_{LR2}) have different lengths of the bend portion (12) in the longitudinal axis direction.
